(19) **Europäisches Patentamt**

**European Patent Office**

**Office européen des brevets**

(11) **EP 1 001 262 B1**

(12) **EUROPEAN PATENT SPECIFICATION**

(45) Date of publication and mention
of the grant of the patent:
**17.11.2004 Bulletin 2004/47**

(51) Int Cl.$^7$: **G01N 27/407**

(21) Application number: **00102029.6**

(22) Date of filing: **20.04.1995**

(54) **Method of measuring a gas component**

Verfahren zum Messen einer Gaskomponente

Méthode pour mesurer un composant de gaz

(84) Designated Contracting States:
**DE FR GB IT**

(30) Priority: **21.04.1994 JP 8306994**
**31.01.1995 JP 1459895**
**08.03.1995 JP 4855195**

(43) Date of publication of application:
**17.05.2000 Bulletin 2000/20**

(60) Divisional application:
**04020248.3**
**04020249.1**

(62) Document number(s) of the earlier application(s) in
accordance with Art. 76 EPC:
**95302646.5 / 0 678 740**

(73) Proprietor: **NGK INSULATORS, LTD.**
**Nagoya-City, Aichi Prefecture 467-8530 (JP)**

(72) Inventors:
- **Kato, Nobuhide, c/o NGK Insulators, Ltd.**
**Nagoya-shi, Aichi-ken (JP)**
- **Nakagaki, Kunihiko, c/o NGK Insulators, Ltd.**
**Nagoya-shi, Aichi-ken (JP)**

(74) Representative: **Paget, Hugh Charles Edward et al**
**Mewburn Ellis LLP**
**York House**
**23 Kingsway**
**London WC2B 6HP (GB)**

(56) References cited:
**US-A- 4 927 517**        **US-A- 5 034 112**
**US-A- 5 049 254**

## Description

The present invention relates to a method for measuring concentration of NOx as a component contained in a measurement gas.

Various measuring methods and devices have been proposed for determining the concentration of a desired gas component in a measurement gas. A known method of measuring NOx in combustion gases, for example, employs a sensor having an oxygen-ion conductive solid electrolyte, such as zirconia, and a Pt-containing electrode and a Rh-containing electrode formed thereon. This method utilizes the ability of Rh to reduce NOx, and determine the NOx concentration by measuring an electromotive force induced between the two electrodes. However, this sensor tends to suffer from noise, since the electromotive force varies to a great extent with a change in the concentration of oxygen contained in the combustion gases, but varies a small extent in response to a change in the NOx concentration. In addition, CO or other reducing gas is needed for the Rh electrode to reduce the NOx. Under a lean combustion condition using an excessively small amount of a fuel, a large amount of NOx is generated, which exceeds the amount of CO generated. Thus, the known sensor is not able to make a measurement with respect to a combustion gas produced under such a lean combustion condition.

There is also known a method of measuring NOx by using a pair of electrochemical pumping cell and sensing cell which include Pt electrodes and oxygen-ion conductive solid electrolyte, and a pair of electrochemical pumping cell and sensing cell which include Rh electrodes and oxygen-ion conductive solid electrolyte, as disclosed in JP-A-63-38154 and JP-A-64-39545. The concentration of NOx is calculated based on a difference in pumping currents flowing through the respective pumping cells. In further methods as disclosed in JP-A-1-277751 and JP-A-2-1543, a first and a second pair of an electrochemical pumping cell and sensing cell are prepared, and a limiting current is measured by a sensor having the first pair of pumping and sensing cells, under the oxygen partial pressure that does not allow reduction of NOx, while a limiting current is measured by a sensor having the second pair of pumping and sensing cells, under the oxygen partial pressure which allows reduction of NOx, so as to measure the NOx concentration based on a difference in the limiting currents of the two sensors. It is also proposed to measure a difference in the limiting currents, using a sensor having a pair of pumping and sensing cells, by regulating the oxygen partial pressure in the measurement gas between two levels, one of which does not allow reduction of NOx while the other allows the NOx reduction.

Referring to Fig. 25 showing the principle of the known methods as described above, a first and a second sensing element 61, 62, which are formed independently of each other, have respective internal spaces 65, 66 which communicate with an exterior measurement-gas space through corresponding diffusion resistance portions 63, 64, and respective electrochemical pumping cells 67, 68 using solid electrolyte. The first sensing element 61 effects pumping of only oxygen under a predetermined diffusion resistance, and the oxygen concentration is obtained by multiplying the pumping current $Ip_1$ by a current sensitivity coefficient $K_1$. The second sensing element 62 having an electrode or catalyst capable of reducing NOx effects pumping of both oxygen and NOx under a predetermined diffusion resistance, and a sum of the oxygen and NOx concentrations is obtained by multiplying the pumping current $Ip_2$ by a current sensitivity coefficient $K_2$. Thus, the NOx concentration "Cu" is calculated according to the following equation:

$$Cn = K_2 \cdot Ip_2 - K_1 \cdot Ip_1$$

In the above method of measuring NOx, however, a considerably small current flows due to NOx to be measured, and the most part of the limiting current is caused by a large amout of oxygen contained in the measurement gas. Therefore, the small current value corresponding to NOx is obtained from a difference between the two large pumping currents $Ip_1$, $Ip_2$. With use of one sensor in which the oxygen partial pressure is regulated as described above, the NOx cannot be continuously measured, and the operating response and measuring accuracy are deteriorated. With use of two sensors having different oxygen partial pressures, a measurement error is likely to occur with a great change in the oxygen concentration of the measurement gas, and therefore this method cannot be employed in automobile applications, for example, where the oxygen concentration in exhaust gases varies to a large extent. This is because the dependency of the pumping current of one of the sensors on the oxygen concentration differs from that of the other sensor. In the case of an automobile running under the air/fuel ratio of 20, for example, the oxygen concentration is generally several percentages of exhaust gases, whereas the NOx concentration is several hundreds of ppm, which is about 1/100 of the oxygen. If the dependency of the pumping current on the oxygen concentration slightly differs between the two sensors, therefore, a difference in the limiting currents due to the varied oxygen concentration becomes larger than a change in the limiting currents due to NOx to be measured. The known method involves other problems. That is, if diffusion means or small leak formed in the pumping cell is clogged with oil ash in the exhaust gases, the pumping current may be undesirably changed, resulting in reduced measuring accuracy. With a great variation in the temperature of the exhaust gases, the measurement results may involve some abnormality. Further, a difference in the chronological changes in the characteristics between the two sensors may lead to measuring errors, making the sensors undurable for use for a long period of time.

While the oxygen present in the measurement gas causes various problems upon measurement of NOx, as described above, the oxygen also causes similar problems, such as reduced measuring accuracy, upon measurement of gas components other than NOx, and these problems have been strongly desired to be solved.

It is therefore a first object of the present invention to provide a method for measuring NOx concentration in a measurement gas, without being affected by the oxygen concentration or its variation, thus assuring improved operating response even in repeated measurements, and improved measuring accuracy for a long period of time.

The present invention may be practiced using an NOx sensor which is able to measure an increased range of NOx, in combustion gases produced under a wide range of the air/fuel ratio, that is, from a rich burn region using excessive fuel to a lean burn region using excessive air.

According to the present invention, there is provided a method of measuring concentration of NOx as a component of a measurement gas, as set out in claim 1.

According to the method of the invention the concentration of NOx can be measured with high accuracy, without being affected by the oxygen concentration or its variation in the measurement gas, assuring improved operating response even in repeated measurements, and improved measuring accuracy for a long period of time. Further, the present method enables measurement of an increased range of NOx, in combustion gases produced under a wide range of the air/fuel ratio, that is, from a rich burn region using excessive fuel to a lean burn region using excessive air, without being affected by oil ash.

The second internal space may be filled with a porous body having the second diffusion resistance.

The oxygen partial pressure in the first internal space is preferably detected, and the oxygen pumping action of the first electrochemical cell is controlled by varying a voltage applied to the first electrochemical cell, based on the detected oxygen partial pressure, so that the oxygen partial pressure in the first internal space is regulated to a constant value.

In a further preferred form of the invention, the second elevated temperature of the second internal space is not lower than the first elevated temperature of the first internal space.

In a still further preferred form of the invention, the oxygen partial pressure in the second internal space is not higher than the oxygen partial pressure in the first internal space.

In the method of the invention, one of the pair of electrodes of the electrochemical cell of the second oxygen pumping means, which is disposed in the second internal space, may serve as the catalyst.

In a preferred form of the method of the invention, the sensing device has a sensing element which includes the first and second oxygen-ion conductive solid electrolytes as integral parts thereof, the first and second internal spaces, the first and second diffusion means and the first and second oxygen pumping means being formed in the sensing element.

The sensing element may have a narrow, flat space having a predetermined diffusion resistance, which space has an opening exposed to the external measurement-gas space, the flat space comprising the first and second diffusion means, the first internal space consisting of a first portion of the flat space adjacent to the opening, in which the first oxygen pumping means is provided, the second internal space consisting of a second portion of the flat space remote from the opening and inside of the first portion, in which the second oxygen pumping means is provided.

Further, the first and second oxygen-ion conductive solid electrolytes may form an oxygen-ion conductive solid electrolyte layer, or two separate oxygen-ion conductive solid electrolyte layers.

The sensing device as described above may further include a porous body filling the opening of the flat space of the sensing element, the porous body having a predetermined diffusion resistance.

In the method of the invention, the sensing device may further include a heater for heating the first and second internal spaces to respective predetermined temperatures. In this case, the sensing device can effectively operates even at a relatively low temperature, while effectively decomposing the gas component of the measurement gas.

In the invention, the first pumping electrode of the electrochemical cell of the second oxygen pumping means, which is disposed in the second internal space, may also serve as the catalyst. This leads to a simplified process of manufacturing the device.

The above-indicated first pumping electrode may be formed of a porous cermet consisting of a ceramic, and a metal which is able to reduce or decompose the gas component having bonded oxygen.

Further, the catalyst may be disposed in the second internal space, in the vicinity of the first pumping electrode of the electrochemical cell of the second oxygen pumping means, or may be superposed on the first pumping electrode of the electrochemical cell of the second oxygen pumping means.

The above-indicated heater may be located nearer to the second internal space than to the first internal space within the sensing element, so that the second internal space is heated to a higher temperature than the first internal space. With the heater thus located, the gas component can be more easily reduced or decomposed in the second internal space.

The above-indicated second diffusion resistance of the second diffusion means is preferably determined to be larger than the first diffusion resistance of the first diffusion means. This effectively eliminates an adverse influence on the measurement of the gas component of the measurement gas, due to clogging by oil ash.

The above and optional objects, features and advantages of the present invention will be better understood by reading the following detailed description of its presently preferred embodiments, when considered in conjunction with the accompanying drawings, in which:

Fig. 1 is a plane view showing a sensing element of an NOx sensor used in one embodiment of the present invention;

Fig. 2 is an elevational view in cross section taken along line A-A of Fig. 1, which shows in enlargement a principal part of the sensing element of Fig. 1;

Fig. 3 is a graph showing a first method of controlling the temperatures of electrodes and the oxygen partial pressures in first and second internal spaces;

Fig. 4 is a graph showing the relationship between the temperature of the sensing element exposed to different exhaust gas temperatures, and the distance from the distal end of the element;

Fig. 5 is a graph showing the relationship between the NO concentration and the pumping current (Ip) obtained by the method of Fig. 3;

Fig. 6 is a graph showing a second method of controlling the electrode temperatures and the oxygen partial pressures in the first and second internal spaces;

Fig. 7 is a graph showing the relationship between the NO concentration and the pumping current (Ip) obtained by the method of Fig. 6;

Fig. 8 is a graph showing a third method of controlling the electrode temperatures and the oxygen partial pressures in the first and second internal spaces;

Fig. 9 is a graph showing a fourth method of controlling the electrode temperatures and the oxygen partial pressures in the first and second internal spaces;

Fig. 10 is a graph showing the relationship between the NO concentration and the pumping current (Ip) obtained by the method of Fig. 9:

Fig. 11 is an elevational view corresponding to that of Fig. 2, showing a sensor used in another embodiment, inwhich an NOx reduction catalyst is superposed on an internal pumping electrode;

Fig. 12 is an elevational view corresponding to that of Fig. 2, showing a sensor used in a further embodiment, in which an oxidation catalyst is provided in a first internal space;

Fig. 13 is an elevational view corresponding to that of Fig. 2, showing another example of providing the oxidation catalyst;

Fig. 14 is an elevational view corresponding to that of Fig. 2, showing a further example of providing the oxidation catalyst;

Fig. 15 is an elevational view corresponding to that of Fig. 2, showing a sensor used in another embodiment having different structures of second internal space and second diffusion means;

Fig. 16 is a graph showing the relationship between the NO concentration and the pumping current (Ip) obtained in the embodiment of Fig. 15;

Fig. 17 is an elevational view corresponding to that of Fig. 2, showing a sensing element as part of another NOx sensor useful in the present invention;

Fig. 18 is an elevational view corresponding to that of Fig. 17, showing a modified example of the sensing element of Fig. 17;

Fig. 19 is a graph showing the relationship between the electromotive force and the oxygen partial pressure at a temperature of 600°C;

Fig. 20 is a graph showing the relationship between the NO concentration and the pumping current detected when different voltages are applied to the first and second electrochemical pumping cells while the first and second internal spaces are kept at 600°C;

Fig. 21 is an elevational view corresponding to that of Fig. 2, showing a sensor used in another embodiment, in which the second internal space and second diffusion means have different structures than those of Fig. 15;

Fig. 22 is a cross sectional view of a sensing element, for explaining the principle of the present invention; and

Fig. 23 is a cross sectional view of a known sensing element, for explaining a known method of measuring a gas component.

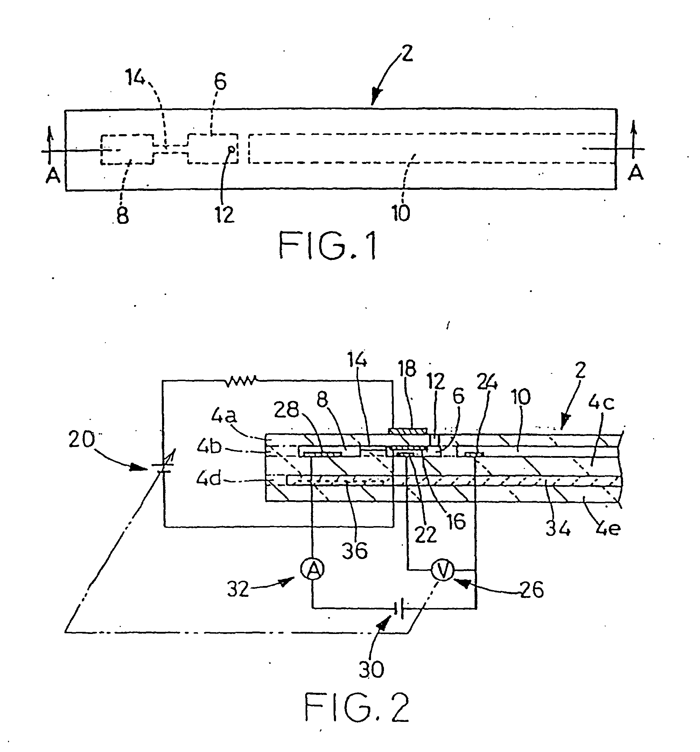

Referring first to Figs. 1 and 2, there is illustrated an NOx sensor used in one preferred embodiment of the method of the present invention, which is adapted to determine the concentration of NOx as a component contained in a measurement gas.

In Figs. 1 and 2, reference numeral 2 denotes an elongate, planar sensing element having a relatively large length. This sensing element 2 has an integral laminar structure including a plurality of highly dense, gastight oxygen-ion conductive solid electrolyte layers 4a, 4b, 4c, 4d, 4e which are superposed on each other, as shown in Fig. 2. Each of the solid electrolyte layers 4a-4e is formed of a known oxygen-ion conductive solid electrolyte material, such as zirconia. The thus integrally formed sensing element 2 is produced by firing unfired solid electrolyte layers into an integral laminar structure, as known in the art.

In the integral sensing element 2, a first internal space 6 and a second internal space 8 are separately formed such that the second internal space 8 is located nearer to the distal end of the sensing element 2.

Each internal space 6, 8 has a rectangular shape as seen in a plane parallel to opposite major surfaces of the element 2. Independently of the first and second internal spaces 6, 8, a reference-gas channel 10 serving as a reference-gas chamber is formed in the longitudinal direction of the sensing element 2, such that the channel 10 is open at the proximal end of the element 2, for communication with the atmosphere. In the instant embodiment, the first and second internal spaces 6, 8 and reference-gas channel 10 are formed in substantial the same plane, by closing respective apertures formed through the solid electrolyte layer 4b, with the upper and lower solid electrolyte layers 4a, 4c. The sensing element 2 further has a first diffusion controlling passage 12 as a first diffusion means, which is formed through the thickness of the upper solid electrolyte layer 4a. The first diffusion controlling passage 12 communicates the first internal space 6 with an exterior space where a measurement gas to be measured by the NOx sensor is present, so that the measurement gas is introduced into the first internal space 6 through the passage 12, under a predetermined diffusion resistance provided by the passage 12. Further, second diffusion means in the form of a second diffusion controlling passage 14 is formed in the solid electrolyte layer 4b, to extend between the first and second internal spaces 6, 8. This second diffusion controlling passage 14 communicates with the first and second internal spaces 6, 8, so that the atmosphere in the first internal space 6 is introduced into the second internal space 8 through the passage 14, under a predetermined diffusion resistance provided by the passage 14.

Inner electrode (pumping electrode) 16, which is a rectangular porous platinum electrode, is formed on the inner surface of the solid electrolyte layer 4a which is exposed to the first internal space 6. Further, outer electrode (pumping electrode) 18, which is also a rectangular porous platinum electrode, is formed on the outer surface of the solid electrolyte layer 4a, such that the inner and outer electrodes 16, 18 are aligned with each other in a plane parallel to the major surfaces of the sensing element 2. These electrodes 16, 18 and solid electrolyte layer 4a constitute an electrochemical cell of first oxygen pumping means, that is, a first electrochemical pumping cell. In operation, a desired voltage is applied between the inner and outer electrodes 16, 18 of the first electrochemical pumping cell, by means of an external variable power supply 20, to generate current flow in a predetermined direction, so that oxygen in the atmosphere in the first internal space 6 is pumped out into the external measurement-gas space, or oxygen is pumped from the external measurement-gas space into the first internal space 6. In this embodiment, each of the porous platinum electrodes 16, 18 consists of a cermet electrode formed of a mixture of platinum and zirconia ($ZrO_2$).

Measuring electrode 22, which is a rectangular, porous platinum electrode, is formed on the surface of the solid electrolyte layer 4c which is exposed to the first internal space 6, while reference electrode 24, which is a similar porous platinum electrode, is formed on the surface of the solid electrolyte layer 4c which is exposed to the the reference-gas channel 10. These measuring electrode 22, reference electrode 24 and solid electrolyte layer 4c constitute an electrochemical cell of oxygen partial pressure detecting means, that is, an electrochemical sensing cell. As known in the art, an electromotive force (EMF) is induced between the measuring electrode 22 and the reference electrode 24, based on a difference in the oxygen concentration between the atmosphere in the first internal space 6 and the reference gas in the reference-gas channel 10. This electromotive force is measured by a potentiometer 26, to thus detect the oxygen partial pressure in the atmosphere in the first internal space 6. Then, a voltage of the variable power supply 20 is regulated or controlled, on the basis of the level of the oxygen partial pressure as detected by the potentiometer 26, whereby the oxygen partial pressure in the atmosphere in the first internal space 6 is kept at a constant level.

Internal pumping electrode (first pumping electrode) 28 is formed on the surface of the solid electrolyte layer 4c, such that the electrode 28 is located within the second internal space 8. This internal pumping electrode 28 is formed of porous cermet consisting of rhodium (Rh) as a metal capable of reducing NOx, and zirconia as a ceramic material. Thus, the electrode 28 also functions as an NOx reduction catalyst which is able to reduce NOx present in the atmosphere in the second internal space 8. When a constant voltage is applied between the internal pumping electrode 28 and the reference electrode (second pumping electrode) 24 disposed in the reference-gas channel 10, by means of a power supply 30, oxygen in the atmosphere within the second internal space 8 is pumped out into the reference-gas channel 10. Thus, the reference electrode 24 also functions as a pumping electrode, and cooperates with the internal pumping electrode 28 and solid electrolyte layer 4c to constitute an electrochemical cell of second oxygen pumping means, that is, a second electrochemical pumping cell. Ammeter 32 is adapted to detect a pumping current which flows by the pumping action of the second oxygen pumping means. The constant voltage applied by the power supply 30 is determined so as to provide a limiting current that enables the second electrochemical pumping cell to effect the pumping of the oxygen generated by reduction of NOx, which is introduced into the second internal space 28, under the diffusion resistance provided by the second diffusion resistance channel 14.

The sensing element 2 also incorporates an alumina insulating layer 34 which is laminated integrally on the solid electrolyte layers 4c, 4e, such that the layer 34 is surrounded by three solid electrolyte layers 4c, 4e and 4d. Heater 36 is embedded within the alumina insulating layer 34, and is operated by an external power supply. As shown in Fig. 2, the heater 36 is located in a distal end portion of the sensing element 2 in which the second internal space 8 is formed, so that the second internal space 8 is heated to a higher temperature than the first internal space 6, in other words, the internal pumping electrode 28 is heated to a higher temperature than the inner electrode 16 and measuring electrode 22. For example, the heater 36 is located so that the inner electrode 16 and measuring electrode 22 in the first internal space 6 is heated up to 400°C-600°C while the internal pumping electrode 28 in the second internal space 8 is heated up to 700°C-900°C, as the temperature of the measurement gas varies in the range from 300°C to 850°C.

The thus constructed sensing element 2 is positioned at its distal end portion within the external measurement-gas space. Thus, the measurement gas is introduced into the first internal space 6, through the first diffusion controlling passage 12, under a predetermined diffusion resistance. Then, the measurement gas in the first internal space 6 is subjected to the oxygen pumping action in which a suitable voltage is applied between the inner and outer electrodes 16, 18 of the first electrochemical pumping cell, so that the oxygen partial pressure in the first internal space 6 is controlled to a predetermined level, for example, $10^{-6}$atm.

To keep the oxygen partial pressure in the atmosphere in the first internal space 6 at the predetermined constant level, a voltage applied between the two electrodes 16, 18 of the first electrochemical pumping cell by the variable power supply 20 is regulated so that the electromotive force induced between the measuring electrode 22 and the reference electrode 24 of the electrochemical sensing cell, which is detected by the potentiometer 26, is equal to 203mV at 500°C, for example, according to the Nernst equation known in the art. In this manner, the oxygen partial pressure in the first internal space 6 can be easily controlled to $10^{-6}$atm as desired. That is, the voltage applied to the first electrochemical pumping cell is regulated so that the above-described electromotive force corresponds to a difference between a desired oxygen concentration in the first internal space 6 and the oxygen concentration of the reference gas. It is to be noted that the first diffusion controlling passage 12 functions to reduce an amount of oxygen in the measurement gas diffusing into the first internal space 6, and thus restrict a current flowing through the first electrochemical pumping cell, upon application of a voltage to the pumping cell.

In the first internal space 6, the oxygen partial pressure is controlled so that NOx contained in the atmosphere is not reduced by the inner electrode 16 and the measuring electrode 22, that is, the reaction: $NO \rightarrow 1/2N_2 + 1/2O_2$, for example, does not take place, even at a high temperature elevated by the external measurement gas and the heater 36. If NOx in the atmosphere is reduced in the first internal space 6, NOx cannot be accurately measured in the second internal space 8, as described later. In this sense, it is necessary to establish appropriate conditions or environment in the first internal space 6 so that NOx is not reduced by a component (at least a component of the inner electrode 16 of the first electrochemical pumping cell), which might otherwise be involved in reduction of NOx in the first internal space 6.

The measurement gas, whose oxygen partial pressure has been controlled in the first internal space 6 in the above manner, is then introduced into the second internal space 8, through the second diffusion controlling passage 14, under a predetermined diffusion resistance. Then, the measurement gas in the second internal space 8 is subjected to an oxygen pumping action, in which a predetermined or constant voltage, e.g., 449mV, is applied at 700°C between the internal pumping electrode 28 and the reference electrode 24 of the second electrochemical pumping cell, so that oxygen is pumped from the second internal space 8 into the reference-gas channel 10. As a result, the oxygen concentration is reduced down to $10^{-10}$atm in the second internal space 8, particularly at the three phase boundary of the internal pumping electrode 28. Consequently, NOx is reduced, that is, the reaction: $NO \rightarrow 1/2N_2 + 1/2O_2$, for example, takes place, around the internal pumping electrode 28 serving as NOx reduction catalyst. At this time, the current flowing through the second electrochemical pumping cell is proportional to the oxygen concentration of the atmosphere in the second internal space 8, that is, a sum of the oxygen concentration of the atmosphere in the first internal space 6 and the concentration of oxygen generated by reduction of NOx at the internal pumping electrode 28. Since the oxygen concentration of the atmosphere in the first internal space 6 is kept a constant, as described above, the current flowing through the second electrochemical pumping cell is proportional to the concentration of NOx. The NOx concentration corresponds to an amount of NOx which is diffused under the resistance of the second diffusion controlling passage 14. In this manner, the measurement of the NOx concentration can be effected.

When the oxygen concentration of the atmosphere in the first internal space 6 is equal to 0.02ppm while the NO concentration of the measurement gas is equal to 100ppm, for example, the current flowing through the second electrochemical pumping cell corresponds to 50.02ppm, that is, a sum of the concentration of oxygen (50ppm) generated by reduction of NO and the oxygen concentration (0.02ppm) of the atmosphere in the first internal space 6. Thus, the pumping current value of the second electrochemical pumping cell substantially represents the amount of NO reduced, and does not depend much on the oxygen concentration of the measurement gas.

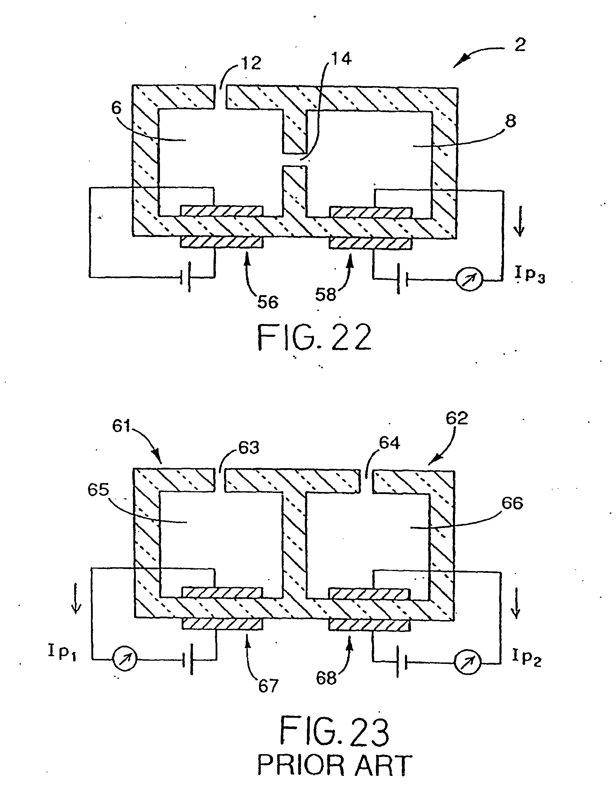

The principle of the present invention will be described in detail, referring to Fig. 22. In this figure, the measurement gas is introduced into the first internal space 6, through the first diffusion controlling passage 12, and the oxygen partial pressure in the first internal space 6 is regulated to a predetermined, desirably low level, by the pumping action of the first electrochemical pumping cell 56 serving as oxygen concentration control means. NOx is not reduced in this first internal space 6. Then, the atmosphere in the first internal space 6, having the thus regulated oxygen pressure, is introduced, through the second diffusion controlling passage 14, into the second internal space 8 where NOx is reduced. The oxygen generated by reduction of NOx is then pumped out from the second internal space 8, by means of the second electrochemical pumping cell 58, and the amount of NOx in the measurement gas is measured on the basis of a magnitude of a current flowing through the second electrochemical cell 58.

In the above-described method, the NOx concentration "Cn" is obtained according to an equation: $Cn = K \cdot Ip_3 - A$, where K is a current sensitivity coefficient, $Ip_3$ is a current flowing through the second electrochemical pumping cell 58, and A is a constant indicative of a small amount of oxygen remaining in the first internal space 6. It will be understood that the most part of $Ip_3$ depends on the oxygen generated by decomposition of the NOx component contained in the measurement gas, and that the present method permits highly accurate measurement of even a slight amount of NOx, without being affected by oxygen in the measurement gas, as compared with conventional methods. The outer electrodes of the first and second electrochemical pumping cells 56, 58, which are not exposed to the internal spaces 6, 8, may be located anywhere provided that oxygen can be discharged from these electrodes. For example, the outer electrodes are located in the ambient air.

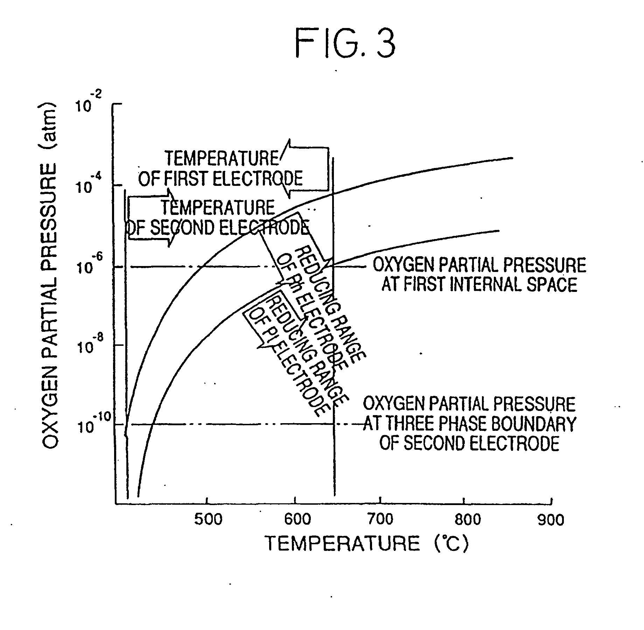

The graph of Fig. 3 shows one example of the relationship between the temperatures at the electrodes in the respective internal spaces 6, 8 and the oxygen partial pressures in these internal spaces 6, 8 which are controlled in the manner as described above. In this graph, the first electrode is a cermet Pt electrode containing $ZrO_2$ and Pt in the volume ratio of 40 : 60, which electrode is co-fired with a $ZrO_2$ substrate at 1400°C, while the second electrode is a cermet Rh electrode containing $ZrO_2$ and Rh in the volume ratio of 40 : 60. This graph also shows the capability of these electrodes to reduce NO in relation to the oxygen partial pressure and the temperature at each internal space. It will be apparent from Fig. 3 that the Pt electrode is only capable of reducing NO at a relatively high temperature, under a relatively low oxygen partial pressure, while the Rh electrode is capable of reducing NO at a relatively low temperature, under a relatively high oxygen partial pressure.

It will be understood from the above-indicated relationship between the oxygen concentration and the temperature that the temperature of the first electrodes, that is, the Pt electrodes (inner electrode 16 and measuring electrode 22) disposed in the first internal space 6, is controlled to within a range in which NO is not reduced by the first electrodes. For example, when the oxygen partial pressure in the first internal space 6 is set to $10^{-6}$ atm, as shown in Fig. 3, the location and power of the heater 36 are determined so that the temperature in the first internal space 8 is equal to or lower than 650°C, even when the temperature of the measurement gas is 900°C (which is approximately the maximum temperature of the exhaust gases of automobiles). The measurement gas whose oxygen partial pressure is controlled to $10^{-6}$atm in the first internal space 6 is then introduced, through the second diffusion controlling passage 14, into the second internal space 8 where a voltage of 450mV is applied to the internal pumping electrode 28 and the reference electrode 24, to pump out oxygen from the second internal space 8 into the reference-gas channel 10. Thus, the oxygen partial pressure at the three phase boundary of the internal pumping electrode 28 is lowered to about $10^{-10}$atm (which corresponds to the electromotive force of 450mV that develops across the internal pumping electrode 28 and the reference electrode 24). It will be noted from Fig. 3 that the second electrode, that is, the Rh electrode as the internal pumping electrode 28, is capable of reducing NO at 410°C or higher, under the oxygen partial pressure of $10^{-10}$atm. Accordingly, the location and power of the heater 36 are determined so that the temperature of the internal pumping electrode 28 (or second electrode) disposed in the second internal space 8 is equal to or higher than 410°C, even when the temperature of the measurement gas is 300°C (which is approximately the lowest temperature of the automobile exhaust gases). In this manner, NO is reduced at the three phase boundary of the internal pumping electrode 28 as the second electrode, to generate oxygen which causes a current to flow through the second electrochemical pumping cell. The magnitude of this current is proportional to the concentration of NO.

[0043] In the above-described first example, the oxygen partial pressure in the first internal space 6 is controlled to $10^{-6}$atm while the oxygen partial pressure in the second internal space 8, more precisely, at the three phase boundary of the internal pumping electrode 28 (second electrode), is controlled to $10^{-10}$atm. At the same time, the location and power of the heater 36 are determined so that the temperature at the inner electrode 16 and the measuring electrode 22 disposed in the first internal space 6 is not higher than 650°C, and the temperature at the internal pumping electrode 28 in the second internal space 8 is not lower than 410°C, as the temperature of the automobile exhaust gases varies over the full range of 300 to 900°C, for example.

While a desired temperature of the sensing element 2 may be achieved by controlling the power of the heater 36, depending upon the temperature of the measurement gas, e.g., exhaust gases, the temperature of the element may be easily controlled as desired by merely applying a suitable voltage to the heater 36, without controlling its power. That is, the sensing element 2 generally has a higher temperature at its distal end portion exposed to the exhaust gases. If the heater 36 is positioned close to the distal end of the element 2, and the second internal space 8 including the internal pumping electrode 28 is provided in the distal end portion while the first internal space 6 including the inner electrode 16 and measuring electrode 22 is located apart from the distal end, the above-indicated desired temperatures of these electrodes can be achieved by applying a suitable voltage to the heater 36.

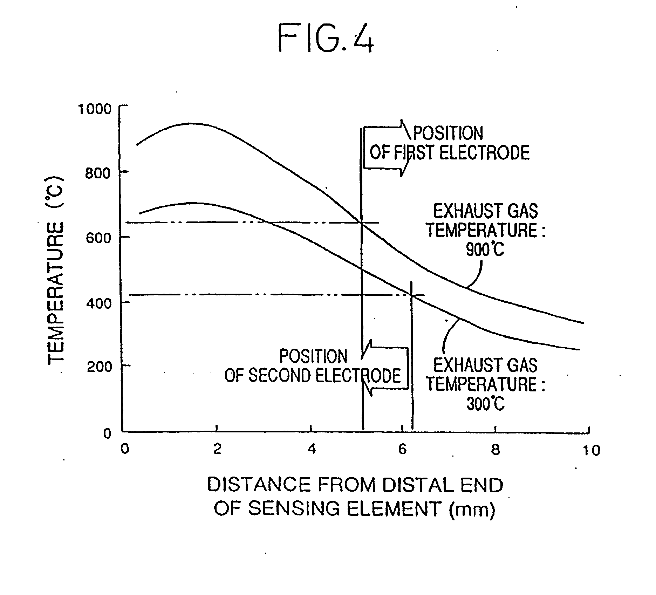

Fig. 4 is a graph showing results of a test for determining the location of the heater in the sensing element. A sensing element used for the test has a platinum heater embedded in a $ZrO_2$ solid electrolyte substrate, at one of various points of the element which range from 1mm to 6mm as measured from its distal end. The $ZrO_2$ substrate has a width of 4.2mm, a thickness of 1.3mm and a length of 62mm, and the platinum heater has a heating portion having a width of 3.6mm and a length of 5mm, and has a resistance of 8Ω at room temperature. With this sensing element installed in an exhaust pipe of an automobile, a voltage of 12V is applied to the platinum heater, and the temperatures at the above-described various points of the element are measured when exposed to exhaust gases having 300°C and 900°C.

As is apparent from Fig. 4, the temperature of the sensing element is 650°C or lower in its region which is 5.2mm or more away from the distal end of the element, when the exhaust gas has the maximum temperature of 900°C. Therefore, the first electrodes, i.e., the inner electrode 16 and measuring electrode 22 are located in the above region that is 5.2mm or more away from the distal end. When the exhaust gas has the lowest temperature of 300°C, the temperature of the sensing element is 410°C or higher in its region which is 0 - 6.2mm away from the distal end of the element. Therefore, the second electrode, i,e, the internal pumping electrode 28, is located in the above region that is in the 0 - 6.2mm distance from the distal end. In this manner, there is obtained the NOx sensor according to the present invention, in which the temperatures of the respective electrodes are controlled as shown in the graph of Fig. 3. It is to be noted that the distribution of the temperatures in the sensing element as described above can be achieved as desired, by suitably selecting the power (resistance), size (length) and location of the heater.

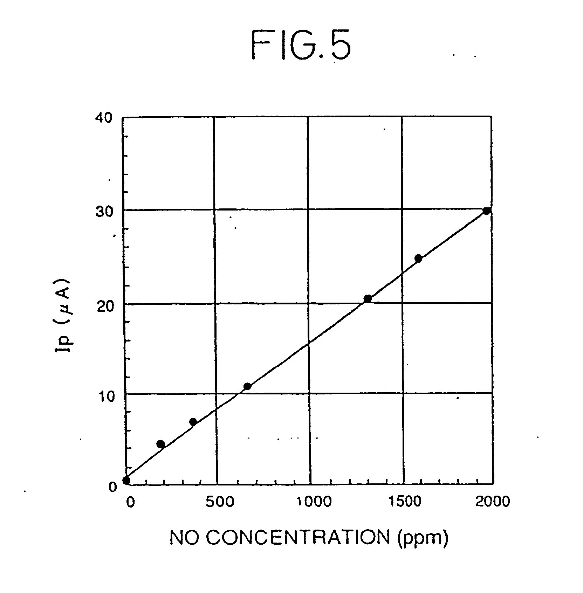

Fig. 5 is a graph showing the relationship between the NO concentration measured by the above-described NOx sensor, and the pumping current (diffusion limiting current: Ip). It is found in this graph that the pumping current (Ip) is linearly proportional to the NO concentration. Thus, the NO concentration can be easily obtained by measuring the current (Ip). In this case, Ip is 0.03μA when NO is equal to 0 ppm, which means that the pumping current of 0.03μA is required to pump out an amount of oxygen which corresponds to a difference between the oxygen concentration ($10^6$atm) in the atmosphere in the first internal space 6, and the oxygen concentration in the atmosphere in the second internal space 8, more precisely, at the three phase boundary of the internal pumping electrode 28. Although this pumping current of 0.03μA does not affect the measurement of the NO concentration of several dozens of ppm, taking account of the sensitivity: 30μA/2000ppm = 0.015μA/ppm, the same pumping current may cause an error when the NO concentration to be measured is lower than 10ppm. It is therefore preferable that the pumping current is as closest to 0 as possible when NO is equal to 0ppm. This can be achieved either by controlling the oxygen partial pressure in the atmosphere in the first internal space 6 tb the possibly lowest level while avoiding reduction of NOx, or by equalizing the oxygen partial pressure in the first internal space 6 with that in the second internal space 6, measured at the three phase boundary of the internal pumping electrode 28.

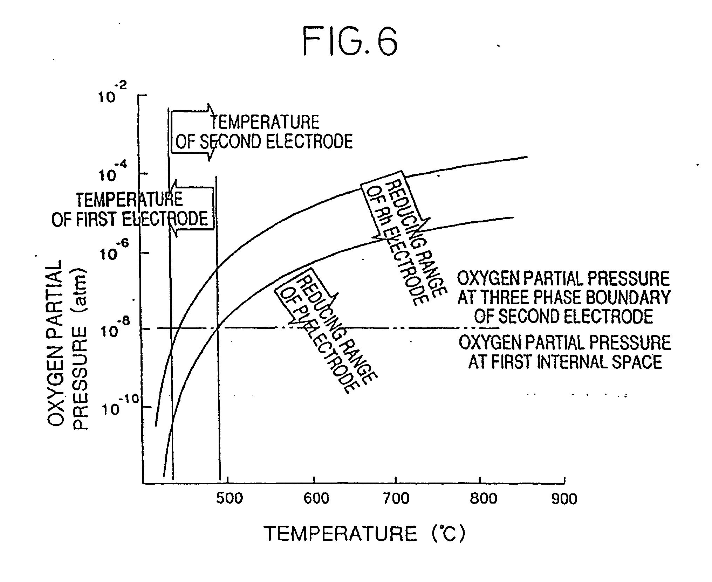

Fig. 6 is a graph showing a second example of the relationship between the temperatures at the electrodes in the first and second internal spaces 6, 8, and the oxygen partial pressures in these internal spaces 6, 8 which are controlled in the manner as described above. In this example, the oxygen partial pressure in the first internal space 6, and the oxygen partial pressure at the three phase boundary of the internal pumping electrode 28 (second electrode) in the second internal space 8 are both controlled to $10^{-8}$ atm. The heater 36 is provided so that the temperature at the first electrodes (inner electrode 16 and measuring electrode 22) is 490°C or lower, and the temperature at the second electrode (internal pumping electrode 28) is 430°C or higher.

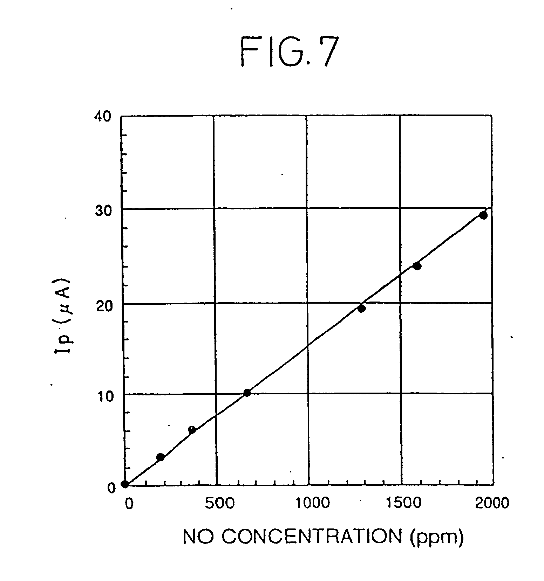

Fig. 7 is a graph showing the relationship between the pumping current (Ip) and the NO concentration measured by the NOx sensor of the second example. Since the oxygen partial pressures in the first internal space 6 and at the three phase boundary of the second electrode (internal pumping electrode 28) in the second internal space 8 are controlled to the same value in this example, Ip is 0μA where NO is equal to 0ppm.

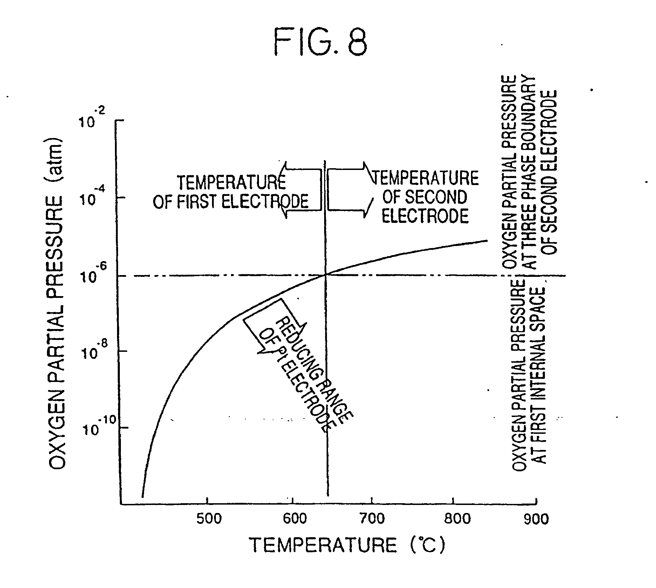

Fig. 8 is a graph showing a third example of the relationship between the temperatures at the electrodes and the oxygen partial pressures. In this example, the first electrodes (inner electrode 16 and measuring electrode 22) and the second electrode (internal pumping electrode 28) are both comprised of Pt electrodes, and the oxygen partial pressures in the first internal space 6 and at the three phase boundary of the second electrode (internal pumping electrode 28) in the second internal space 8 are both controlled to $10^{-6}$atm. The heater 36 is provided so that the temperature at the first electrodes is lower than 650°C, and the temperature at the second electrode is 650°C or higher.

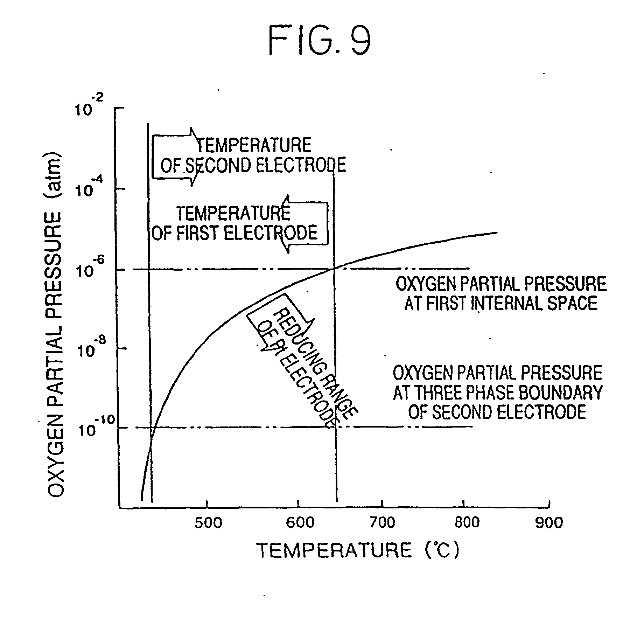

Fig. 9 is a graph showing a fourth example of the relationship between the temperatures at the electrodes and the oxygen partial pressures. In this example, the first electrodes (inner electrode 16 and measuring electrode 22) and the second electrode (internal pumping electrode 28) are both comprised of Pt electrodes, and the oxygen partial pressure in the first internal space 6 is controlled to $10^{-6}$ atm. while the oxygen partial pressure at the three phase boundary of the second electrode (internal pumping electrode 28) in the second internal space 8 is controlled to $10^{-10}$ atm. The heater 36 is provided so that the first electrodes are heated to 650°C or lower, and the second electrode is heated up to 430°C or higher.

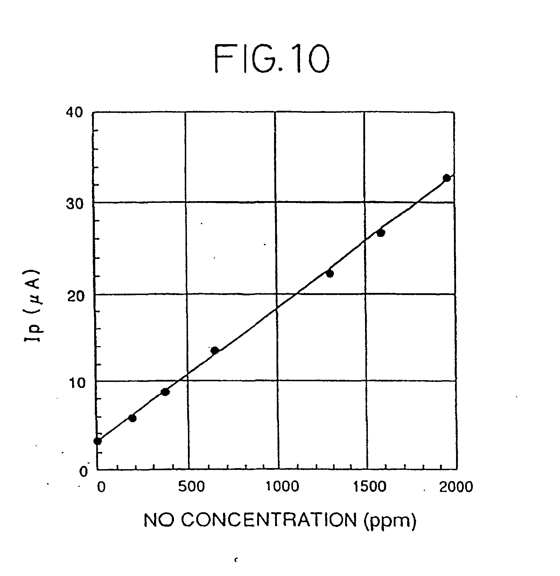

In the fourth example, a pumping current $IP_0$ flows when NO is equal to 0ppm, as described above. This pumping current $IP_0$ is kept a constant if the oxygen partial pressures in the first and second internal spaces 6, 8 are kept constant, and can therefore be easily subtracted from the pumping current Ip. Fig. 10 is a graph showing the relationship between the pumping current Ip and the NO concentration measured by the NOx sensor of this example.

In the above-described examples, the measurement gas is introduced into the first internal space, where the oxygen concentration of the gas is controlled to a predetermined value by the oxygen pumping action of the first electrochemical pumping cell, while the oxygen partial pressure and the temperature are suitably controlled to avoid reduction of NOx. Then, the measurement gas having the predetermined oxygen concentration is introduced from the first internal space into the second internal space, where the temperature and oxygen concentration are determined so that the NOx component in the gas is reduced at the three phase boundary of the NOx reduction catalyst (internal pumping electrode 28) disposed in the second internal space. A pumping current measured upon the oxygen pumping action of the second electrochemical pumping cell is proportional to the NOx concentration of the measurement gas. Thus, NOx can be effectively determined on the basis of the pumping current, without being affected by the oxygen concentration of the measurement gas.

In the above-described NOx sensor , the first internal space 6 is positioned downstream of the first diffusion controlling passage 12, and the second diffusion controlling passage 14 is positioned downstream of the first internal space 6, as seen in the direction of flow of the measurement gas. Therefore, clogging due to oil ash may take place at the first diffusion controlling passage 12, but not likely at the second diffusion controlling passage 14. If the diffusion resistance values D1, D2 of the first and second diffusion controlling passages 12, 14 are determined to satisfy the relationship: D1 + $\alpha$ « D2, where $\alpha$ is a variation of the diffusion resistance due to clogging, the measurement of the NOx concentration is not affected by such clogging. Thus, the clogging of the first diffusion controlling passage 12 only results in reduction of the pumping current for keeping the oxygen concentration in the first internal space at a constant level, and will not affect the measurement of NOx since the gas containing NOx is substantially diffused through the second diffusion controlling passage 14.

In the first and second examples, the first electrodes (16, 22) disposed in the first internal space 6 are cermet electrodes containing Pt, while the second electrode (28) is a cermet electrode containing Rh. In the third and fourth examples, the first and second electrodes are both comprised of cermet electrodes containing Pt. However, the electrode materials are not necessarily limited to those as employed in the above examples. For example, the first electrode may be a cermet electrode containing Au or an alloy of Au and Pt, and the second electrode may be a Rh-containing cermet electrode. Since the Au/Pt cermet electrode as the first electrode is less likely to reduce NOx, the oxygen partial pressure and temperature in the first internal space can be more freely determined from within increased allowable ranges. While metals used for the electrodes may be suitably selected from known metals, both the first and second electrodes may be Au-containing electrodes, with a Rh- or Pt-containing electrode or a catalyst superposed on the second electrode. The catalyst may be a ceramic porous layer formed of alumina, which loads thereon an NOx reducing metal. Alternatively, the first and second electrodes may be Pt-containing electrodes, with a Rh-containing catalyst electrode disposed on the second Pt electrode. It is also possible that the first and second electrodes made of the same material are exposed to different temperatures, for example.

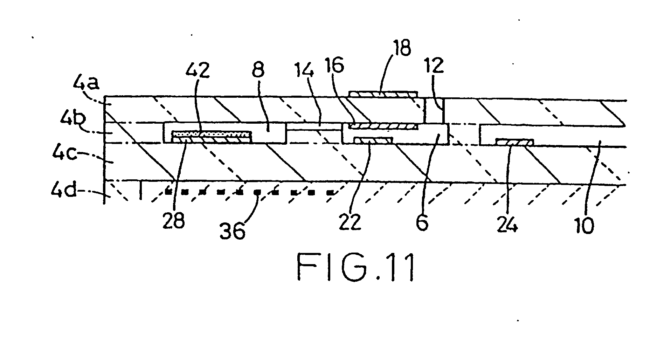

It is desirable that the first and second electrodes are cermet electrodes comprised of a metal and a suitable ceramic material. For the second electrode which function as an NOx reduction catalyst, as in the illustrated embodiment, a porous cermet electrode is desirably employed which is formed of a ceramic and a known metal, such as Rh and Pt, capable of reducing NOx. An NOx reduction catalyst may be provided in the vicinity of the internal pumping electrode 28 disposed in the second internal space 8, or an NOx reduction catalyst 42 as shown in Fig. 11 may be laminated by printing or other method on the internal pumping electrode 28. This catalyst 42 is a porous alumina layer loading an NOx reducing catalyst, such as rhodium. In the sensing element as shown in Fig. 11, the heater 36 is located on the side of the second internal space 8, so that the second internal space 8 is heated to a higher temperature than the first internal space 6, whereby the NOx reduction catalyst 42 performs its function more effectively.

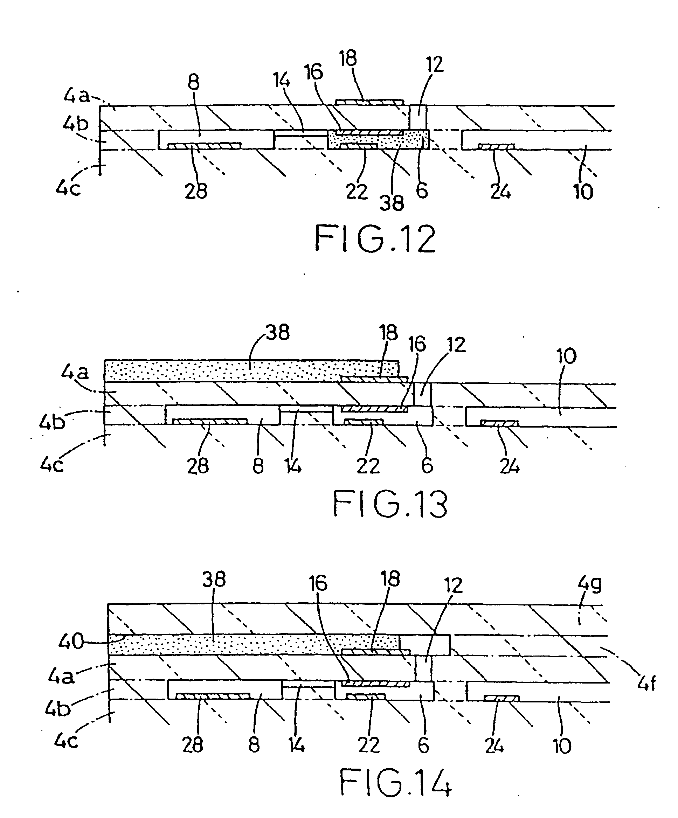

Where the measurement gas consists of exhaust gases generated under a rich burn engine operation, the gas contains large amounts of unburned components, such as CO and HC, which may react with NOx to cause a measuring error of the NOx sensor. To avoid this, the first internal space 6 is preferably filled with an oxidation catalyst 38 formed from porous alumina, for example, for oxidizing the unburned components, such as CO and HC, in the measurement gas, as shown in Fig. 12. In this case, the polarity of the first electrochemical pumping cell is reversed with respect to that in the case of a lean burn engine operation. That is, oxygen in the measurement gas is pumped from the exterior space into the first internal space 6. The provision of the oxidation catalyst 38 in the first internal space 6 is effective for eliminating influences due to the reducing gases, such as CO and HC, even when the measurement gas is produced as a result of the rich burn operation.

While the first internal space 6 is filled with the oxidation catalyst 38 in the embodiment of Fig. 12, the catalyst 38 may be provided on a portion of the solid electrolyte layer 4c between the first diffusion controlling passage 12 and the measuring electrode 22, or may be provided by printing on the inner electrode 16. That is, the oxidation catalyst 38 may be located anywhere provided the unburned components, such as CO and HC, are oxidized before the measurement gas reaches the second internal space 8.

To oxidize the unburned components, such as CO and HC, there is no need to provide the oxidation catalyst 38. That is, the oxidation can be promoted if the inner electrode 16 functions as an oxidation catalyst. Even if the inner electrode 16 consists of an Au or Au/Pt alloy electrode which does not serve as an oxidation catalyst, the unburned components in the measurement gas can be oxidized under appropriate conditions, including the oxygen partial pressure and the temperature, in the first internal space 6. These conditions must be also advantageous for measurement of NOx, assuring the oxygen partial pressure which is as low as possible and closest to that of the second internal space 8. For example, the oxidation can readily take place in the first internal space 6 if the oxygen partial pressure is at least $10^{-10}$ atm at 500°C, and at least $10^{-15}$ atm at 600°C.

In the above-described method of measuring NOx, the measurement gas is controlled in the first internal space 6, to provide an atmosphere which is effective for measurement of NOx. More specifically, oxygen is pumped out from or pumped into the atmosphere in the first internal space 6, by the pumping action of the first electrochemical cell, so that the oxygen partial pressure in the space 6 is kept at a constant level which is almost the same as that in the second internal space 8, and which enables oxidation of the unburned components, such as CO and HC, contained in the measurement gas. Consequently, the oxidation of the unburned components is effected, thereby to avoid reaction between such unburned components and NOx, assuring more accurate measurement of the NOx concentration. This is particularly effective with respect to a measurement gas which is produced as a result of combustion under a rich burn engine operation, and thus contains considerably large amounts of carbon monoxide, hydrocarbon and other unburned components.

The oxidation of the unburned components in the measurement gas as described above is effective for eliminating any influence by the unburned components on the NOx measurement accuracy, with respect to exhaust gases produced not only under the rich burn engine operation, but also under the lean burn engine condition which may produce slight amounts of such unburned components as described above.

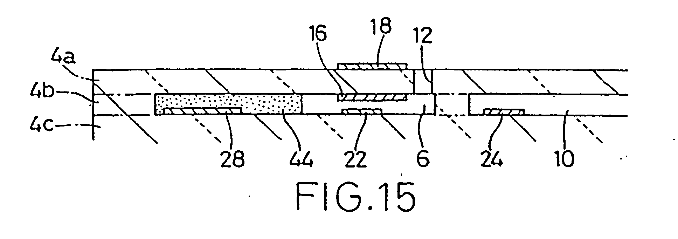

The oxidation catalyst 38 may be laminated on the upper solid electrolyte layer 4a, as shown in Fig. 13, such that the catalyst 38 is located in the external measurement gas space while closing an open end of the first diffusion controlling passage 12. This is effective mainly for eliminating measuring errors due to CO and HC produced when the engine operates under lean A/F condition. This embodiment is not suitable for the rich burn engine operation since the whole amounts of CO and HC cannot be oxidized due to shortage of oxygen in the measurement gas. In a further embodiment as shown in Fig. 14, additional solid electrolyte layers 4f and 4g are superposed on the solid electrolyte layer 4a, with a gas inlet channel 40 defined by these three solid electrolyte layers 4f, 4g, 4a. The oxidation catalyst 38 is located in the gas inlet channel 40. In this case, the oxidation of CO, HC and others is more effectively promoted since the oxidation catalyst 38 is located in a relatively high-temperature distal end portion of the sensor, as compared with the embodiment of Fig. 12.

To regulate the oxygen concentration at the three phase boundary of the second electrode (28) serving as an NOx reduction catalyst, in the second internal space 8, the oxygen is pumped from the second electrode (28) to the reference electrode 24 in the illustrated embodiments. However, the second electrode (28) may cooperate with the outer electrode 18 of the first electrochemical pumping cell, to constitute a second electrochemical pumping cell, so as to pump the oxygen in the second internal space 8 toward the outer electrode 18. It is also possible to provide another electrode in the second internal space 8 to pump out oxygen in the second internal space 8, in addition to the second electrode (28) as the NOx reduction catalyst. In this case, on the basis of an electromotive force induced between the NOx reduction catalyst electrode (28) and the reference electrode 24, oxygen may be pumped from the additional pump-out electrode toward the reference electrode, the outer electrode 18, or a further electrode adapted to pump out the oxygen, so as to regulate the oxygen concentration at the three phase boundary of the NOx reduction catalyst electrode.

In the illustrated embodiments, the oxygen partial pressure in the first internal space 6 is regulated by continuously varying a voltage to be applied to the pumping electrodes 16, 18 of the first electrochemical pumping cell, based on the level of the electromotive force detected at the electrochemical sensing cell. However, a constant voltage may be applied to these pumping electrodes 16, 18, or a single electrochemical cell may serve as both a pumping cell and a sensing cell at different shared time.

The reference electrode 24 is not necessarily held in communication with the atmosphere through the reference-gas channel 10. Rather, the reference electrode 24 may be disposed in a space in which the oxygen that is pumped out from the second electrode (28) is stored.

Further, the second internal space and the second diffusion means may be constituted by a space which is filled with a porous body. More specifically, the second internal space 8 and the second diffusion controlling passage 4 of the sensing element 2 shown in Fig. 2 may be replaced by an arrangement as shown in Fig. 15, in which an internal space adjacent to the first internal space 6 is filled with a porous body 44 made of alumina, for example, to provide both the second diffusion means and the second internal space. This arrangement simplifies the internal structure of the NOx sensor. In this embodiment, the diffusion resistance of the second diffusion means (44) is determined to be larger than that of the first diffusion controlling passage 12, such that the atmosphere in the first internal space 6 is not influenced by the atmosphere in the second internal space.

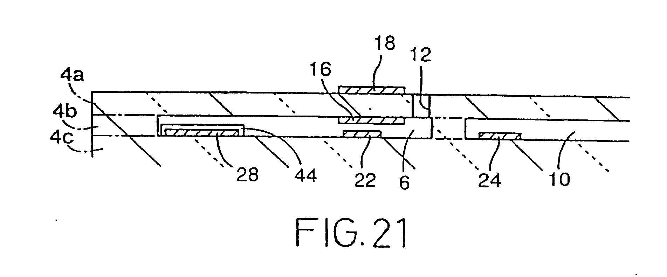

The porous body 44 may be printed on the internal pumping electrode 28, as shown in Fig. 21. In this arrangement, the porous body 44 constitutes the second diffusion means, and the porous body 44 itself substantially provides the second internal space.

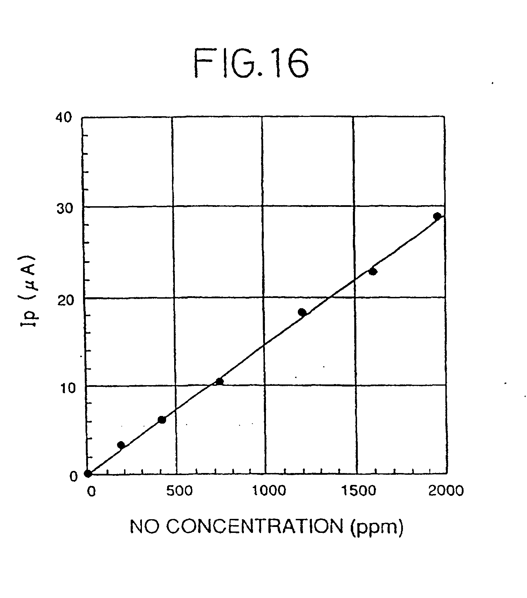

In operation of the sensing element 2 constructed as described above, a voltage applied between the two electrodes 16, 18 of the first electrochemical pumping cell is controlled so that an electromotive force of 203mV is induced at 500°C between the measuring electrode 22 in the first internal space 6, and the reference electrode 24. At the same time, a constant voltage is applied between the internal pumping electrode 28 and the reference electrode 24 of the second electrochemical pumping cell, so that an electromotive force of 449mV is induced at 700°C between these electrodes 28, 24. In this condition, the NO concentration linearly varies with the pumping current (Ip) of the second electrochemical pumping cell, as shown in the graph of Fig. 16. Accordingly, the NO concentration is determined by measuring the magnitude of the pumping current (Ip).

The arrangement of the sensing element as shown in Fig. 15 may be modified such that the porous structure of the second diffusion means 44 loads therein an NOx reduction catalyst, or such that an oxidation catalyst for oxidizing the unburned components, such as CO and HC, is disposed in the first internal space 6. Thus, the present sensing element may be modified as needed, while assuring NOx detecting characteristics similar to those of the sensing element of Fig. 15.

The materials for the electrodes (16, 22) disposed in the first internal space 6 and the electrode (28) disposed in the second internal space (second diffusion means 44) are suitably selected such that the electrode (28) in the second internal space has the same or higher capability of reducing NOx than the electrodes (16, 22) in the first internal space 6. In addition, an NOx reduction catalyst layer may be provided on the electrode (28) in the second internal space.

The location of the reference electrode and the the reference-gas chamber and the kind of the reference gas are suitably selected, such that the electromotive force determined according to the Nernst equation is induced between the measuring electrode in the first internal space 6 and the reference electrode.

The position and power of the heater are suitably selected such that the temperature at the electrodes in the first internal space 6 is lower than the temperature at the electrode in the second internal space (second diffusion means 44).

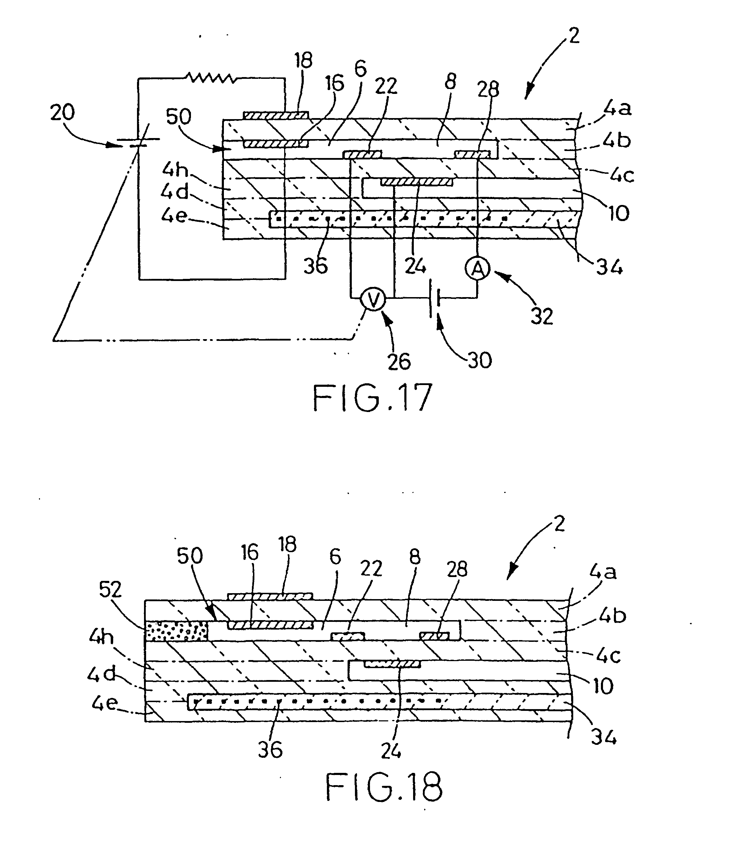

The NOx sensor used in the present invention may have the first and second diffusion means in the form of a narrow, flat space having a predetermined diffusion resistance. The flat space is formed in the sensing element so as to be open to the external measurement-gas space, as shown in Fig. 17 by way of example.

The sensing element 2 as shown in Fig. 17 has an integral laminar structure including six oxygen-ion conductive solid electrolyte layers 4a, 4b, 4c, 4h, 4d, 4e which are superposed on each other in this order. The second uppermost solid electrolyte layer 4b has a rectangular cutout or notch at its distal end portion, which provides a narrow, flat space 50 having a predetermined diffusion resistance. The flat space 50 is open at the distal end of the sensing element 2, and extends over a suitable length in the longitudinal direction of the element 2. That is, the flat space 50 has an elongate, rectangular shape as seen in a plane parallel to the major surfaces of the sensing element 2, and is open at one of the opposite short sides to the external measurement-gas space.

In operation, the external measurement gas is introduced through the opening of the flat space 50, and reaches an innermost part of the flat space 50, under a predetermined diffusion resistance. Thus, the flat space 50 itself constitutes the first and second diffusion means. The inner electrode (pumping electrode) 16 of the first electrochemical pumping cell is provided in a portion of the flat space 50 adjacent to its opening, which portion forms the first internal space 6, while the internal pumping electrode 28 of the second electrochemical pumping cell is provided in a deeper portion of the flat space 50 inside of the first internal space 6, which portion forms the second internal space 8.

In the instant embodiment, reference-gas channel 10 is formed through the solid electrolyte layer 4h, such that the channel 10 is open at a proximal end of the sensing element, for communication with the atmosphere. Reference electrode 24 disposed in the reference-gas channel 10 cooperates with the measuring electrode 22 provided in the flat space 50 to constitute the electrochemical sensing cell, and cooperates with the internal pumping electrode 28 to constitute the second electrochemical pumping cell. Thus, the reference electrode 24 also serves as one of the pumping electrodes.

The other parts of the NOx sensor as shown in Fig. 17 are similar to those of the NOx sensor of Fig. 2, and the same reference numerals are used in the figures for the corresponding elements, of which no detailed description will be provided. The specific structures for the flat space 50 and the reference-gas channel 10 formed in the sensing element 2 are shown in detail in Figs. 10 and 11 of JP-B2-5-18059, from which the structure of the sensing element of Fig. 17 is to be understood.

Fig. 18 shows a modified structure of the sensing element 2 as shown in Fig. 17. An open end portion of the flat space 50 is filled with a porous body 52 having a predetermined diffusion resistance. The measurement gas is introduced through the porous body 52 into the flat space 50, under the predetermined diffusion resistance, and the introduced gas is subjected to an oxygen pumping action of the first oxygen pumping means, in the first internal space 6 provided by a portion of the flat space 50 adjacent to the porous body 52. The provision of the porous body 52 is advantageous in that the diffusion of the gas into the first internal space 6 can be controlled with high reliability, and that unburned components, such as CO and HC, are effectively oxidized at the porous body 52.

In the illustrated embodiments of the method, the effective decomposition and reduction of NOx are achieved due to a temperature difference between the first and second internal spaces 6, 8. However, the atmospheres in the first and second internal spaces 8 do not necessarily have different temperatures. In the example of Fig. 3, for instance, if the temperature of both of the first and second internal spaces 6, 8 is set to 600°C, the Pt electrode (16) disposed in the first internal space 6 will not reduce NOx under the oxygen partial pressure of about $10^{-6}$atm or higher, while the Rh electrode (28) disposed in the second internal space 8 will reduce NOx under the oxygen partial pressure of $10^{-5}$atm or lower. It is therefore possible to measure NOx with high accuracy even when both the first and second internal spaces 6, 8 have the same temperature of 600°C, if the oxygen partial pressure of the atmosphere in the first internal space 6 is controlled to $10^{-6}$atm or higher, and the oxygen partial pressure of the atmosphere in the second internal space 8 is controlled to $10^{-5}$atm or lower.

If the electrodes having no or little capability of reducing NOx, such as those containing Au or an alloy of Au and Pt, are employed, the measurement of NOx can be effected under a condition where the temperature of the first internal space is higher than that of the second internal space. For example, the alloy electrode containing Pt and 1% of Au, which does not reduce NOx at 800°C under the oxygen partial pressure of $10^{-15}$atm, may be employed for measurement of NOx, even if the first internal space is kept at 800°C with the oxygen par-

tial pressure of $10^{-10}$atm, and the second internal space is kept at 600°C with the oxygen partial pressure of $10^{-10}$atm.

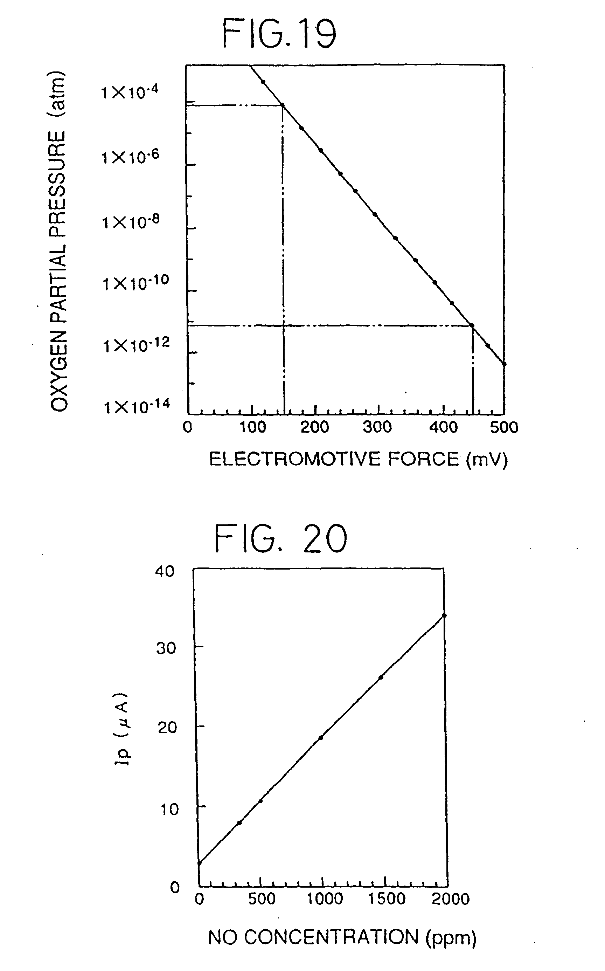

Fig. 19 is a graph showing the relationship between the electromotive force and the oxygen partial pressure at a temperature of 600°C. If the oxygen partial pressure of the atmosphere in the first internal space 6 is regulated so that the electromotive force at the measuring electrode 22 of the electrochemical sensing cell is 150mV, the oxygen partial pressure in the first internal space 6 becomes equal to about $10^{-4}$atm, and therefore NOx is not reduced in the first internal space 6. If 450mV is applied to the internal pumping electrode 28 of the second electrochemical pumping cell, which electrode is disposed in the second internal space 8, the oxygen partial pressure at the three phase boundary of the pumping electrode 28 becomes equal to about $10^{-11}$atm, and therefore NOx is reduced at the internal pumping electrode 28. The oxygen generated by the reduction of NOx can be detected by the pumping current of the second electrochemical pumping cell. The graph of Fig. 20 shows the relationship between the NO concentration and the pumping current (Ip).

## Claims

1. A method of measuring concentration of NOx as a component of a measurement gas, comprising the steps of:

   (i) introducing the measurement gas containing the NOx component from an external measurement-gas space into a first internal space (6), with a first diffusion resistance;
   (ii) controlling the amount of oxygen in the measurement gas within said first internal space so as to produce an atmosphere having a predetermined oxygen partial pressure;
   (iii) introducing said atmosphere from said first internal space into a second internal space (8) with a second diffusion resistance;
   (iv) determining the concentration of the NOx component present in the atmosphere in said second internal space by contacting the gas in said second internal space with a NOx decomposition catalyst so as to derive oxygen from the NOx component and measuring the oxygen in said second internal space; and
   (v) controlling the temperatures of the atmospheres in said first and second internal spaces so that the determined concentration of the NOx component of the atmosphere in said second internal space corresponds to the concentration of NOx in said measurement gas,

   wherein step (iv) comprises:

pumping out oxygen in the second internal space (8) by an oxygen pumping action of an electrochemical cell (4c, 24, 28; 58); and detecting a pumping current flowing through said electrochemical cell (4c, 24, 28; 58); and

wherein the NOx concentration Cn in the measurement gas is obtained on the basis of said pumping current according to the equation:

$$Cn = K \cdot Ip_3 - A$$

where

K is a current sensitivity coefficient,
$Ip_3$ is current flowing through the electrochemical cell (4c, 24, 28; 58), and
A is a constant indicative of the small amount of oxygen remaining in said first internal space (6).

2. A method according to claim 1, wherein said NOx component in said second internal space (8) whose concentration is determined consists essentially of NO.

3. A method according to claim 1 or 2, wherein said amount of oxygen in the measurement gas is controlled in said first internal space (6) by an oxygen pumping action of a first electrochemical cell (4a, 16, 18; 56).

4. A method according to any one of claims 1 to 3, wherein said second internal space (8) is filled with a porous body (44) having said second diffusion resistance.

5. A method according to claim 1, wherein the oxygen partial pressure in said first internal space (6) is regulated by an oxygen pumping action of a first electrochemical pumping cell (4a, 16, 18; 56), so as to produce an atmosphere having a predetermined level of the oxygen partial pressure, which does not allow conversion of NO to $N_2$ and $O_2$ at a first elevated temperature of the first internal space, while the oxygen partial pressure in said second internal space (8) is regulated by pumping out oxygen by the oxygen pumping action of said second electrochemical pumping cell (4c, 24, 28; 58), so that the oxygen partial pressure is regulated to a predetermined level which allows reduction or decomposition of NOx at a second elevated temperature of the second internal space.

6. A method according to claim 3, wherein the oxygen partial pressure in said first internal space (6) is detected, and the oxygen pumping action of said first electrochemical cell (4a, 16, 18; 56) is controlled by varying a voltage applied to the first electrochemical cell, based on the detected oxygen partial pressure, so that the oxygen partial pressure in the first internal space is regulated to a constant level.

7. A method according to claim 1, wherein the oxygen pumping action of said second electrochemical cell (4c, 24, 28; 58) is effected by applying thereto a constant voltage which provides a diffusion limiting current to the gas component, the oxygen partial pressure of the atmosphere in said second internal space (8) being regulated by application of said diffusion limiting current.

8. A method according to claim 5, wherein said second elevated temperature of the second internal space is not lower than said first elevated temperature of the first internal space.

9. A method according to claim 5, wherein the oxygen partial pressure in said second internal space is not higher than the oxygen partial pressure in said first internal space.

10. A method according to claim 6, wherein oxygen is pumped out from the atmosphere in said first internal space due to said oxygen pumping action of said first electrochemical pumping cell, so that the oxygen partial pressure of the atmosphere is made lower than that of the measurement gas.

**Patentansprüche**

1. Verfahren zum Messen der Konzentration von NOx als eine Komponente eines Messgases, folgende Schritte umfassend:

(i) das Einführen des die NOx-Komponente enthaltenden Messgases von einem äußeren Messgas-Raum in einen ersten inneren Raum (6) mit einem ersten Diffusionswiderstand;
(ii) das Steuern der Sauerstoffmenge im Messgas innerhalb des ersten inneren Raums, so dass eine Atmosphäre mit einem vorbestimmten Sauerstoff-Partialdruck erzeugt wird;
(iii) das Einführen der Atmosphäre vom ersten inneren Raum in einen zweiten inneren Raum (8) mit einem zweiten Diffusionswiderstand;
(iv) das Ermitteln der Konzentration der in der Atmosphäre im zweiten inneren Raum enthaltenen NOx-Komponente, indem das Gas im zweiten inneren Raum mit einem NOx-Zersetzungskatalysator kontaktiert wird, um Sauerstoff aus der NOx-Komponente zu erhalten, und der Sauerstoff im zweiten inneren Raum gemessen wird;
(v) das Steuern der Temperaturen der Atmosphären im ersten und im zweiten inneren Raum, so dass die ermittelte Konzentration der NOx-Komponente der Atmosphäre im zweiten inneren Raum der Konzentration von NOx im Messgas entspricht;

worin Schritt (iv) Folgendes umfasst:

das Abpumpen von Sauerstoff im zweiten inneren Raum (8) durch Sauerstoff-Pumpwirkung einer elektrochemischen Zelle (4c, 24, 28; 58); und
das Detektieren eines durch die elektrochemische Zelle (4c, 24, 28; 58) fließenden Pumpstroms; und

worin die NOx-Konzentration Cn im Messgas auf Basis des Pumpstroms gemäß der folgenden Gleichung erhalten wird:

$$Cn = K.Ip_3 - A$$

worin K ein Stromempfindlichkeitskoeffizient ist, $Ip_3$ der durch die elektrochemische Zelle (4c, 24, 28; 58) fließende Strom ist, und A eine Konstante ist, die die geringe Menge an im ersten inneren Raum (6) verbleibendem Sauerstoff angibt.

2. Verfahren nach Anspruch 1, worin die NOx-Komponente im zweiten inneren Raum (8), deren Konzentration ermittelt wird, im Wesentlichen aus NO besteht.

3. Verfahren nach Anspruch 1 oder 2, worin die Sauerstoffmenge im Messgas im ersten inneren Raum (6) durch Sauerstoff-Pumpwirkung einer ersten elektrochemischen Zelle (4a, 16, 18; 56) gesteuert wird.

4. Verfahren nach einem der Ansprüche 1 bis 3, worin der zweite innere Raum (8) mit einem porösen Körper (44) gefüllt ist, der den zweiten Diffusionswiderstand aufweist.

5. Verfahren nach Anspruch 1, worin der Sauerstoff-Partialdruck im ersten inneren Raum (6) durch Sauerstoff-Pumpwirkung einer ersten elektrochemischen Pumpzelle (4a, 16, 18; 56) gesteuert wird, so dass eine Atmosphäre mit einem vorbestimmten Sauerstoff-Partialdruck erzeugt wird, der keine Umwandlung von NO in $N_2$ und $O_2$ bei einer ersten erhöhten Temperatur des ersten inneren Raums zulässt, während der Sauerstoff-Partialdruck im zweiten inneren Raum (8) durch Abpumpen von Sauerstoff durch die Sauerstoff-Pumpwirkung der zweiten elektrochemischen Pumpzelle (4c, 24, 28; 58) so reguliert wird, dass der Sauerstoff-Partialdruck auf ein vorbestimmtes Maß eingestellt wird, das eine Reduktion oder Zersetzung von NOx bei einer zweiten erhöhten Temperatur des zweiten inneren Raums zulässt.

6. Verfahren nach Anspruch 3, worin der Sauerstoff-Partialdruck im ersten inneren Raum (6) detektiert wird und die Sauerstoff-Pumpwirkung der ersten elektrochemischen Zelle (4a, 16, 18; 56) durch Variieren einer an die erste elektrochemische Zelle angelegten Spannung auf Basis des detektierten Sauerstoff-Partialdrucks so gesteuert wird, dass der Sauerstoff-Partialdruck im ersten inneren Raum auf einem konstanten Wert gehalten wird.

7. Verfahren nach Anspruch 1, worin die Sauerstoff-Pumpwirkung der zweiten elektrochemischen Zelle (4c, 24, 28; 58) dadurch bewirkt wird, dass eine konstante Spannung an diese angelegt wird, die für die Gaskomponente einen Diffusionsgrenzstrom bereitstellt, wobei der Sauerstoff-Partialdruck der Atmosphäre im zweiten inneren Raum (8) durch Anlegen des Diffusionsgrenzstroms reguliert wird.

8. Verfahren nach Anspruch 5, worin die zweite erhöhte Temperatur des zweiten inneren Raums nicht niedriger als die erste erhöhte Temperatur des ersten inneren Raums ist.

9. Verfahren nach Anspruch 5, worin der Sauerstoff-Partialdruck im zweiten inneren Raum nicht höher als der Sauerstoff-Partialdruck im ersten inneren Raum ist.

10. Verfahren nach Anspruch 6, worin Sauerstoff aus der Atmosphäre im ersten inneren Raum aufgrund der Sauerstoff-Pumpwirkung der ersten elektrochemischen Pumpzelle abgepumpt wird, so dass der Sauerstoff-Partialdruck der Atmosphäre niedriger wird als jener des Messgases.

**Revendications**

1. Procédé pour mesurer la concentration des oxydes d'azote en tant que composant d'un gaz de mesure, comprenant les étapes consistant à

(i) introduire le gaz de mesure contenant le composant NOx d'un espace de gaz de mesure externe dans un premier espace interne (6), avec une première résistance à la diffusion ;
(ii) régler la quantité d'oxygène dans le gaz de mesure dans le premier espace interne de façon à produire une atmosphère ayant une pression partielle d'oxygène prédéterminée ;
(iii) introduire ladite atmosphère dudit premier espace interne dans un second espace interne (8) avec une seconde résistance à la diffusion ;

(iv) déterminer la concentration du composant NOx présent dans l'atmosphère dans ledit second espace interne en mettant en contact le gaz dans ledit second espace interne avec un catalyseur de décomposition des NOx de manière à obtenir de l'oxygène à partir du composant NOx, et mesurer l'oxygène dans ledit second espace interne, et

(v) régler les températures des atmosphères dans lesdits premier et second espaces internes de façon que la concentration déterminée du composant NOx de l'atmosphère dans ledit second espace interne corresponde à la concentration des NOx dans ledit gaz de mesure,

où l'étape (iv) comprend :

le pompage de l'oxygène hors du second espace interne (8) par une action de pompage d'oxygène d'une cellule électrochimique (4c, 24, 28 ; 58) ; et
détecter un courant de pompage s'écoulant à travers ladite cellule électrochimique (4c, 24, 28 ; 58) ; et

où la concentration Cn des NOx dans le gaz de mesure est obtenue sur la base dudit courant de pompage en accord avec l'équation :

$$Cn = K.Ip_3 - A$$

où

K est un coefficient de sensibilité du courant,
$Ip_3$ est le courant passant à travers la cellule électrochimique (4c, 24, 28 ; 58), et
A est une constante indicative de la petite quantité d'oxygène restant dans ledit premier espace interne (6).

2. Procédé selon la revendication 1, où ledit composant NOx dans ledit second espace interne (8), dont la concentration est déterminée, est constitué essentiellement de NO.

3. Procédé selon la revendication 1 ou 2, où ladite quantité d'oxygène dans le gaz de mesure est réglée dans ledit premier espace interne (6) par une action de pompage d'oxygène d'une première cellule électrochimique (4a, 16, 18 ; 56).

4. Procédé selon l'une des revendications 1 à 3, où ledit second espace interne (8) est rempli par un corps creux (44) ayant ladite seconde résistance à la diffusion.

5. Procédé selon la revendication 1, où la pression partielle de l'oxygène dans ledit premier espace interne (6) est réglée par une action de pompage d'oxygène d'une première cellule de pompage électrochimique (4a, 16, 18 ; 56) de manière à produire une atmosphère présentant un niveau prédéterminé de la pression partielle de l'oxygène, qui ne permet pas la conversion de NO en $N_2$ et $O_2$ à une première température élevée du premier espace interne, tandis que la pression partielle de l'oxygène dans ledit second espace interne (8) est réglée en pompant l'oxygène, par l'action de pompage d'oxygène, hors de ladite seconde cellule de pompage électrochimique (4c, 24, 28 ; 58) de sorte que la pression partielle de l'oxygène est réglée à un niveau prédéterminé qui permet la réduction ou la décomposition des NOx à une seconde température élevée du second espace interne.

6. Procédé selon la revendication 3, où la pression partielle de l'oxygène dans ledit premier espace interne (6) est détectée, et l'action de pompage de l'oxygène de ladite première cellule électrochimique (4a, 16, 18 ; 56) est commandée en faisant varier une tension appliquée à ladite première cellule électrochimique, sur la base de la pression partielle détectée de l'oxygène de telle sorte que la pression partielle de l'oxygène dans le premier espace interne est réglée à un niveau constant.

7. Procédé selon la revendication 1, où l'action de pompage d'oxygène de ladite seconde cellule électrochimique (4c, 24, 28 ; 58) est effectuée en appliquant à celle-ci une tension constante qui fournit un courant limitant la diffusion au composant gazeux, la pression partielle de l'oxygène de l'atmosphère dans ledit second espace interne (8) étant réglée en appliquant ledit courant limitant la diffusion.

8. Procédé selon la revendication 5, où ladite seconde température élevée du second espace interne n'est pas plus basse que ladite première température élevée du premier espace interne.

9. Procédé selon la revendication 5, où la pression partielle de l'oxygène dans ledit second espace interne n'est pas plus élevée que la pression partielle de l'oxygène dans ledit premier espace interne.

10. Procédé selon la revendication 6, où l'oxygène est pompé hors de l'atmosphère dans ledit premier espace interne par ladite action de pompage d'oxygène de ladite première cellule de pompage électrochimique de sorte que la pression partielle de l'oxygène de l'atmosphère est rendue plus basse que celle du gaz de mesure.

FIG.1

FIG.2

# FIG. 3

# FIG.4

TEMPERATURE (°C) vs DISTANCE FROM DISTAL END OF SENSING ELEMENT (mm)

- POSITION OF FIRST ELECTRODE
- POSITION OF SECOND ELECTRODE
- EXHAUST GAS TEMPERATURE: 900°C
- EXHAUST GAS TEMPERATURE: 300°C

# FIG.5

# FIG.6

# FIG.7

# FIG. 8

# FIG. 9

# FIG.10

FIG.11

FIG.12

FIG.13

FIG.14

FIG.15

# FIG.16

FIG.17

FIG.18

# FIG.19

# FIG. 20

FIG. 21

FIG. 22

FIG. 23
PRIOR ART